# EUROPÄISCHE PATENTSCHRIFT

(11) **EP 2 084 128 B1**
(45) Veröffentlichungstag und Bekanntmachung des Hinweises auf die Patenterteilung: **14.05.2014**
(21) Anmeldenummer: 07821518.3
(22) Anmeldetag: 18.10.2007
(51) Int. Cl.: C07C 263/10, B01J 19/00

(54) **VERFAHREN ZUR HERSTELLUNG VON ISOCYANATEN**
PROCESS FOR PREPARING ISOCYANATES
PROCÉDÉ DE PRÉPARATION D'ISOCYANATES

(30) Priorität: 26.10.2006 EP 06123015
(43) Veröffentlichungstag der Anmeldung: 05.08.2009
(73) Patentinhaber: BASF SE, 67056 Ludwigshafen (DE)
(72) Erfinder: BÖHLING, Ralf, 64653 Lorsch (DE); KÜBER, Sven, 67169 Kallstadt (DE); MAIXNER, Stefan, 68723 Schwetzingen (DE); STROEFER, Eckhard, 68163 Mannheim (DE); HENKELMANN, Jochem, 68165 Mannheim (DE); KRUG, Georg, 69509 Mörlenbach (DE); BOLZ, Werner, 67071 Ludwigshafen (DE)
(86) Internationale Anmeldenummer: PCT/EP2007/061153
(87) Internationale Veröffentlichungsnummer: WO 2008/049783

(56) Entgegenhaltungen:
- WO-A1-01/91898
- DE-A1- 10 026 142
- W. EFRENFELD, V. HESSEL, H. LÖWE: "Microreactors: New Technology for Modern Chemistry" 2000, WILEY-VCH , WEINHEIM , XP002465177 Kapitel 1, "State of the Art of Microreaction Technology" Seite 1 - Seite 14

## Beschreibung

Die Erfindung betrifft ein bevorzugt kontinuierliches Verfahren zur Herstellung von Isocyanaten, bevorzugt Di und/oder Polyisocyanaten, durch Phosgenierung von Aminen, bevorzugt Di- und/oder Polyaminen, bevorzugt in flüssiger Phase, wobei man Phosgen und Amin in mindestens 2, bevorzugt mindestens 10, besonders bevorzugt mindestens 100, Insbesondere bevorzugt zwischen 100 und 100 000, Insbesondere zwischen 100 und 20 000 parallel geschalteten Mischkammern in Kontakt bringt, wobei man als Mischkammern mikrostrukturierte Mischkammern einsetzt.

Die Synthese von Isocyanaten, die wichtige Grundprodukte für die Herstellung von Polyurethanen sind, erfolgt großtechnisch durch die Phosgenierung des entsprechenden Amins. Die Ausbeuten liegen hierbei bei über 90% bezogen auf das eingesetzte Amin. Diese Umsetzung ist allgemein bekannt und vielfältig beschrieben.

Die Phosgenierung in flüssiger Phase, die insbesondere bei der Herstellung von MDI in Frage kommt, wird beschrieben in DE-A 17 68 439, DE-A 198 04 915 und EP-A 1 073 628. Ansätze zur Lösung der bei der Flüssigphasenphosgenierung auftretenden Feststoffprobleme durch Bildung von Aminhydrochloriden, Harnstoffen, Carbamylchloriden werden in EP-A 792 263, WO 01/91898 und WO 03/99770 beschrieben.

Die Gasphasenphosgenierung als Alternative zur Phosgenierung in flüssiger Phase wurde z.B. zur Herstellung von HDI und TDI beschrieben in EP-A 570799, EP-A 699657 und EP-A 593334. Problematisch bei der Umsetzung in der Gasphase sind die notwendigen hohen Temperaturen, die zu unerwünschten Nebenprodukten führen können und bei den erheblichen Umsätzen in großtechnischen Anlagen gerade bei exothermen Reaktionen nur schwer konstant gehalten werden können. Zudem ist die Gasphasenphosgenierung gerade für die Herstellung von MDI ausgehend vom Roh-MDA, das auch höhere Homologe enthält, nicht umsetzbar.

DE-A 100 26 142 offenbart ein Verfahren und eine Vorrichtung zur kontinuierlichen Herstellung von organischen Mono- oder Polyisocyanaten in einer Mischkammer, in der die Eduktströme nach dem Gegenstromprinzip aufeinandertreffen.

Ehrfeld et al. (Ehrfeld, W., Hessel, V., Löwe, H. Microreactors - New Technology for Modem Chemistry, WILEY-VCH, 2000) beschreiben den Aufbau und die Funktionsweise von Mikroreaktoren sowie deren Verwendung in der chemischen Synthese.

Nachteilig an den bekannten Verfahren, die alle auf gegebenenfalls mehrstufige Einstrangverfahren bezogen sind, sind somit die auftretenden Feststoffprobleme durch Nebenprodukte.

Aufgabe der vorliegenden Erfindung war es somit, ein Verfahren zur Herstellung von Isocyanaten, insbesondere MDI zu entwickeln, bei dem insbesondere feste Nebenprodukte möglichst vermieden werden. Diese lassen sich zwar in einem gewissen Umfang durch eine Erhöhung des Phosgenüberschusses lösen, doch besteht eine weitere Aufgabe darin, den Phosgen-hold-up in der Anlage, d.h. die in der Anlage vorhandene Phosgenmenge zu minimieren. Des weiteren sollte das erhältliche Isocyanat ein möglichst gutes Eigenschaftsprofil aufweisen.

Diese Aufgaben konnte durch ein Verfahren zur Herstellung von Isocyanaten, bevorzugt Di und/oder Polyisocyanaten, besonders bevorzugt MDI und TDI, durch Phosgenierung von Aminen, bevorzugt Di- und/oder Polyaminen, besonders bevorzugt MDA und TDA, gelöst werden, bei dem man Phosgen und Amin in mindestens 2, bevorzugt mindestens 10, besonders bevorzugt mindestens 100, insbesondere bevorzugt zwischen 100 und 100 000, insbesondere zwischen 100 und 20 000 parallel geschalteten, bevorzugt räumlich getrennten Mischkammern in Kontakt bringt, wobei man als Mischkammern mikrostrukturierte Mischkammern einsetzt.

Das erfindungsgemäße Verfahren zeichnet sich dadurch aus, das die Vermischung der Edukte in mehreren, räumlich getrennten und parallel angeordneten Kammern erfolgt und bevorzugt die Stoffströme an späterer Stelle bevorzugt zur weiteren Aufarbeitung wieder vereinigt werden. Diese auf den ersten Blick aufwendige Anordnung mit bevorzugt einer Vielzahl von parallelen Stoffströmen bietet den Vorteil, dass aufgrund der größeren Reaktoroberfläche auch höhere Reaktionstemperaturen sehr gut kontrolliert und eingestellt werden können. Aufgrund der damit technisch umsetzbaren höheren Temperaturen können unerwünschte Nebenprodukte wie Aminhydrochloride, Harnstoffe, Carbamylchloride unterdrückt oder vermieden werden. Zudem werden durch die möglichen höheren Temperaturen kürzere Verweilzeiten und damit eine Reduzierung des Reaktorvolumens mit einem entsprechend geringeren Phosgen-hold-up erzielbar.

Durch die im Vergleich zur bekannten Phosgenierung in flüssiger Phase erfindungsgemäß mögliche höhere Reaktionstemperatur können die bekannten Nachteile der Flüssigphasenphosgenierung nämlich insbesondere Feststoffbildung sowie lange Nachreaktionszeiten vermieden werden.

Das erfindungsgemäße Verfahren zeichnet sich somit auch durch eine Verringerung der Verweilzeit in der Reaktionszone aus, wobei eine geringere Verweilzeit zu besserer Produktqualität führt und bedingt durch die Apparategröße einen im Vergleich zum Stand der Technik geringeren Phosgen- und Lösemittel-hold-up zulässt.

In dem erfindungsgemäßen Verfahren stellen die Mischkammern primär die Punkte dar, an denen das Amin mit dem Phosgen in Kontakt gebracht wird. Dabei handelt es sich bei den Mischkammern aber bevorzugt nicht nur um reine Mischeinrichtungen, sondern diese Mischkammern sind bereits Teil des Reaktionsraumes oder stellen den Reaktionsraum dar, in dem die Phosgenierung primär stattfindet. Unter dem Ausdruck Reaktionsraum wird dabei bevorzugt der Raum verstanden, in dem das Reaktionsgemisch die bevorzugte Reaktionstemperatur aufweist. Dabei können die parallelen Stoffströme der verschiedenen Mischkammern bereits innerhalb des Reaktionsraumes zu einem Strom vereinigt werden. Alternativ ist es auch möglich, das Reaktionsgemisch vor einer Vereinigung der Stoffströme abzukühlen und erst anschließend zu vereinigen und aufzuarbeiten.

Erfindungsgemäß werden somit In einer Mehrzahl von Mischkammern Phosgen und Amin in Kontakt gebracht. Dabei wird man bevorzugt in jede der Mischkammern das Phosgen und das Amin durch getrennte Zuläufe zuführen. Dies bedeutet, dass jede Mischkammer mindestens einen Zulauf für Phosgen und mindestens einen Zulauf für das Amin aufweist. Dabei können die einzelnen Zuläufe für jede Mischkammer durch eine einzige Zuleitung von Phosgen bzw. Amin gespeist werden. Dies bedeutet, dass jeweils eine Zuführung für Phosgen und eine für das Amin aufgespalten werden in einzelne Zuleitungen für die einzelnen Mischkammern, deren Stoffströme wiederum an späterer Stelle vereinigt und gemeinsam aufgearbeitet werden. Bevorzugt kann man somit einen Phosgenstrom und einen Aminstrom jeweils in eine Mehrzahl von Einzelströmen aufspalten und die Einzelströme den Mischkammern zuführen, wobei jede Mischkammer mindestens einen Phosgenzulauf und mindestens einen Aminzulauf aufweist.

Das Volumen der einzelnen Mischkammer beträgt zwischen 0,01 cm³ und 1 1, bevorzugt zwischen 0,01 und 10 cm³, besonders bevorzugt zwischen 0,01 und 1 cm³. Dabei wird unter dem Volumen der Mischkammer das Volumen verstanden, dass die einzelne Mischkammer zwischen dem Ort des Zusammentreffens des Amins mit dem Phosgen und dem Ort, an dem dieser Stoffstrom mit dem Stoffstrom einer parallel geschalteten, weiteren Mischkammer zusammentrifft, aufweist. Dabei wird das Volumen des Raumes mitgerechnet, an dem das Amin mit dem Phosgen zusammentrifff.

Die parallele Anordnung von mehreren Mischkammern bietet wie eingangs dargestellt den Vorteil, dass im Vergleich zu einer Anlage gleicher Kapazität mit einem einzigem Stoffstrom die einzelnen, parallelen Stoffströme kleiner sind und damit thermisch besser kontrolliert werden können. Es werden als Mischkammer mikrostrukturierte Mischkammern eingesetzt. Mikrostrukturierte Mischkammern sind allgemein bekannt und vielfältig beschrieben. Eine allgemeine Darstellung über mikrostrukturierte Mischkammern befindet sich in Chemical Micro Process Engineering, V. Hessel et al., 2004, 200-214.

Diese mikrostrukturierten Mischkammern bieten in besonderem Maße den Vorteil, dass aufgrund der großen Oberfläche der Mischkammer bzw. des Reaktionsraumes im Verhältnis zum Volumen eine besonders gute und sichere Temperaturführung erreicht werden kann.

Bevorzugt setzt man als Mischkammer laminare Diffusionsmischer, Multilaminationsmischer, Mikromischer mit strukturierten Wänden, Split-Recombine-Mischer, Freistrahlmischer und/oder Düsen ein, besonders bevorzugt werden T- bzw. Y-Mischer eingesetzt.

Bei den laminaren Diffusionsmischern erfolgt die Vermischung von Teilströmen des Fluids, das an einer Mikrostruktur in eine Vielzahl mikroskopisch kleiner Strömungslamellen mit einer Dicke im Bereich von 10 bis 2.000 µm, oder auch 20 bis 1.000 µm oder auch 40 bis 500 µm aufgefächert wurde, ausschließlich durch molekulare Diffusion senkrecht zur Hauptströmungsrichtung. Eine überschlagsmäßige Auslegung des Mischers kann über die Fourier-Zahl Fo = τ/τ_{D} erfolgen. Liegt die Verweilzeit τ mindestens in der Größenordnung der Diffusionszeit τ_{D} für die transversale. Vermischung, d. h. besitzt die Fourier-Zahl mindestens den Wert 1, erzielt man am Ausgang des Mischers nahezu vollständige molekulare Vermischung.

Laminare Diffusionsmischer können als einfache T- oder Y-Mischer oder als so genannte Multilaminationsmischer ausgeführt sein. Beim T- oder Y-Mischer werden die beiden zu mischenden Teilströme durch eine T- oder Y-förmige Anordnung einem Einzelkanal zugeführt. Maßgebend für den transversalen Diffusionsweg S_{Diff} ist hierbei die Kanalweite δ_{K}. Für typische Kanalweiten zwischen 100 µm und 1 mm ergeben sich für Gase sehr kleine Mischzeiten von weniger als 100 ms, wohingegen diese bei Flüssigkeiten im Minutenbereich liegen. Im Fall des Mischens von Flüssigkeiten oder überkritischen Fluiden, wie im Falle des vorliegenden Verfahrens, ist es vorteilhaft, den Mischvorgang zusätzlich, beispielsweise durch strömungsinduzierte Quervermischung, zu unterstützen.

Bei Multilaminatiorismischern werden die zu vermischenden Teilströme in einem Verteiler in eine Vielzahl von Stromfäden geometrisch vereinzelt und am Austritt des Verteilers dann alternierend in Lamellen der Mischstrecke zugeführt. Bei Flüssigkeiten erreicht man mit den klassischen Multilaminationsmischern Mischzeiten im Sekundenbereich. Da dies für manche Anwendungen (z. B. bei schnellen Reaktionen) nicht ausreichend ist, wurde das Grundprinzip dahingehende weiterentwickelt, dass die Strömungslamellen nochmals zusätzlich geometrisch oder hydrodynamisch fokussiert werden. Bei der geometrischen Fokussierung geschieht dies durch eine Verengung in der Mischstrecke und bei der hydrodynamischen Fokussierung durch zwei Seitenströme, die den Hauptstrom senkrecht anströmen und so die Strömungslamellen weiter komprimieren. Durch die beschriebene Fokussierung lassen sich laterale Abmessungen der Strömungslamellen von wenigen Mikrometern realisieren, so dass selbst Flüssigkeiten innerhalb von einigen 10 ms gemischt werden können.

Bei Mikromischern mit strukturierten Wänden sind sekundäre Strukturen auf den Kanalwänden angeordnet, beispielsweise Riefen oder Stege, in einem bestimmten Winkel zur Hauptströmrichtung, bevorzugt von 45° oder 90°.

Split-Recombine-Mischer zeichnen sich durch Stufen aus wiederkehrender Trennung und Zusammenführung von Strömen aus. Bei jeder dieser Stufen wird die Lamellenzahl sukzessive verdoppelt und dadurch Lamellendicke und Diffusionsweg halbiert.

Bei Düsen erfolgt die Mischung der Edukte in einer Mischzone ohne bewegte Einbauten unter Ausnutzung der vorhandenen oder sich ausbildenden Turbulenz. Die Vermischung sollte zur Vermeidung von Nebenprodukten in einer Zeit kleiner 1s bevorzugt kleiner 0,1 s erfolgen. Die beiden Eduktströme Phosgen und Amin, letzterer gegebenenfalls verdünnt mit einem inerten Lösungsmittel, werden der Mischzone separat zugeführt. Der Eintritt der Eduktströme in die Mischzone kann über jeweils einen oder mehrere Eintrittsquerschnitte beliebiger Form erfolgen. Bevorzugt sind dabei kreisförmige, elliptische, kreisringförmige und rechteckige Querschnitte. Die Hauptströmungsrichtung in der Mischzone kann mit den Hauptströmungsrichtungen der zugeführten Eduktströme in den Eintrittsquerschnitt beliebige Winkel zwischen 0° (parallele Zuführung) bis 180° (Gegenstromanordnung) einschließen. Die eintretenden Eduktströme können vor dem Eintritt in die Mischzone beschleunigt werden. Die Mischzone kann eine geometrisch beliebig geformter Raum sein. Bevorzugt ist es ein rotationssymmetrischer oder spiegelsymmetrisches Volumen welches mit Ausnahme der Zuführungsund Ableitungsquerschnitte durch feste Wände begrenzt ist. In einer Ausführungsform (Koaxialdüse) erfolgt die Zuführung des Aminstroms über einen kreisförmigen Querschnitt, während das Phosgen über einen Kreisring zugeführt wird. Die Anordnung der Eintrittsquerschnitte ist dabei koaxial mit der Achse der zylinderförmigen Mischzone. Die Normale auf dem Eintrittsquerschnitt des Amins bildet mit der Zylinderachse einen Winkel von 0°. Die Normale auf dem Eintrittsquerschnitt des Phosgenstroms bildet mit der Zylinderachse einen Winkel von 0 bis 90°. In einer weiteren alternativen Ausführungsform (Ringspaltdüse) erfolgt die Zuführung des Aminstroms, gegebenenfalls verdünnt mit einem inerten Lösungsmittel, über einen Ringspalt. Dieser ist koaxial mit der ebenfalls kreisringförmigen oder kreisförmigen Mischzone angeordnet. Die Zuführung des Phosgenstroms erfolgt über zwei, ebenfalls koaxial zur Mischzonenachse angeordnete Ringspalte, wobei diese beiden Ringspalte den Ringspalt für die Aminzuführung einschließen. In einer weiteren alternativen Ausführung wird der Aminstrom zentral und koaxial einer rotationssymmetrischen Mischzone zugeführt. Die Zuführung des Phosgenstroms erfolgt über einen oder mehrere Querschnitte, die auf dem äußeren Umfang der Mischzone verteilt sind und deren Normalen mit der Achse der Mischzone einen Winkel von 60 bis 90° einschließen.

Die Mischkammern können aus für diesen Zweck allgemein bekannten gegenüber dem Reaktionsgemisch korrosionsfesten Materialien gefertigt sein, z.B. Hasteloy (HC4, HB2), Titan, Tantal, Glas, Keramik. Besonderes bevorzugt können die Mischkammern auf Hasteloy und/oder Glas basieren.

Der Mischzone schließt sich ein Reaktionsraum an, in dem die reaktive Umsetzung der vermischten Komponenten erfolgt. Der Reaktionsraum kann beliebiger geometrischer Form sein. Bevorzugt sind rotationssymmetrische und spiegelsymmetrische Volumina. Der Querschnitt des Reaktionsraums (senkrecht zur Hauptströmungsrichtung) kann über die Länge konstant, erweiternd oder verengend sein.

Wie bereits eingangs dargestellt umfasst der Ausdruck "Mischkammer" die parallel angeordneten Stoffströme zwischen dem in Kontakt bringen von Amin und Phosgen bis zur Vereinigung mit einer der parallelen Mischkammern. Bevorzugt wird man somit nach dem Mischen des Phosgens mit dem Amin in den Mischkammern die Stoffströme der einzelnen Mischkammern zusammenführen und bevorzugt gemeinsam aufarbeiten.

Die Umsetzung des Amins mit dem Phosgen erfolgt bevorzugt bei einer Temperatur von größer als 180°C, bevorzugt zwischen 220 °C und 300 °C, insbesondere zwischen 230 °C und 270 °C. Die Umsetzungstemperatur wird somit bevorzugt nahe oder höher als die Temperatur des Beginns der Dissoziation der Hydrochloride der angewandten Amine gewählt. Diese bevorzugte Reaktionstemperatur definiert auch den Ausdruck "Reaktionsraum" in dieser Schrift als den Raum, in dem das Reaktionsgemisch diese Temperatur aufweist. Die Temperierung des Reaktionsgemisches kann beispielsweise dadurch erfolgen, dass man durch Strukturen, z.B. Kanäle, die sich nahe (z.B. gegenüberliegend) der Reaktionszone befinden, durch die ein Wärmeträgermedium geleitet wird, Wärme zu bzw. abführt. Besonders bevorzugt erfolgt die Wärmeabfuhr über die Wärmeleitung des Materials des Reaktionsapparates, so kann z.B. die Wärme aus dem Reaktionsraum über Wärmeleitung in den Eintrittsbereich der Edukte geführt werden und an diese abgegeben werden. Die Edukte weisen hierfür bevorzugt die entsprechende Eintrittstemperatur auf. Bei Reaktionen hoher Exothermie kann die Wärmeableitung durch allgemeine bekannte Maßnahmen unterstützt werden.

Bevorzugt wird man das Amin mit einer Temperatur von größer als 180°C, bevorzugt größer als 230 °C, besonders bevorzugt zwischen 230 °C und 300 °C, insbesondere zwischen 230 °C und 270 °C in die Mischkammern einführen. Dabei kann man das Amin besonders bevorzugt in Mischung mit einem für diesen Zweck allgemein bekannten inerten organischem Lösungsmittel, bevorzugt Benzol, Toluol, Xylol, Dichlorbenzol, Monochlorbenzol, bevorzugt mit einem Gewichtsanteil zwischen 5 und 90 Gew.-%, bezogen auf das Gesamtgewicht von Amin und Lösungsmittel, in die Mischkammern einführen.

Bevorzugt wird man Amin und Phosgen in einem molaren Verhältnis von Amin zu Phosgen zwischen 1 : 2 und 1 : 20, besonders bevorzugt zwischen 1 : 4 und 1 : 13 den Mischkammern zuführen. D.h. die Umsetzung erfolgt bei einem entsprechenden Überschuss an Phosgen bezogen auf das Amin.

Das Phosgen kann man bevorzugt mit einer Temperatur größer als 180°C, besonders bevorzugt zwischen 220°C und 300°C, insbesondere zwischen 240°C und 270°C in die Mischkammern einführen.

Durch die Kombination hoher Temperatur mit hoher Phosgenkonzentration im Umsetzungsgemisch wird die Dauer der Isocyanatherstellung stark verkürzt sowie die Bildungvon Nebenprodukten (wie salzsauren Aminen und substituierten Harnstoffen) unterdrückt und die Ausbeuten an sekundären Nebenprodukten verringert. Hierdurch wird die Herstellung verschiedenster Mono-, Di- und Polyisocyanate aus Aminen und Phosgen in hoher Ausbeute in einer einzigen Temperaturstufe und in einem einzigen Durchgang durch den Reaktor ermöglicht.

Die Umsetzung des Amins mit dem Phosgen wird man bevorzugt bei einem Druck zwischen 20 und 100 bar, besonders bevorzugt zwischen 30 bar und 60 bar, durchführen. Der Druck wird somit bevorzugt in Abhängigkeit von den Eigenschaften des verwendeten Lösungsmittels gewählt und ist bevorzugt gleich oder höher als der zum Halten aller Bestandteile des Reaktionsgemisches in flüssiger Phase erforderliche Druck.

Der Verbleib der Ausgangsstoffe in der heißen Reaktionszone übersteigt in einer bevorzugten Ausführungsform in der Regel nicht 5 bis 60 Sekunden, dabei ist die Dauer von der Natur des Amins abhängig. Besonders bevorzugt ist die Verweilzeit der Reaktionsmischung bei einer Temperatur von größer als 180°C, bevorzugt zwischen 220 °C und 300 °C, insbesondere zwischen 230 °C und 270 °C kleiner als 60 s, bevorzugt zwischen 0,01 und 30 s, besonders bevorzugt zwischen 0,1 s und 2 s. Dabei kann die Verweilzeit wie in der DE-A 17 68 439 angegeben bestimmt werden.

Im Anschluss an die reaktive Umsetzung wird man zur Vermeidung der Bildung von Neben- und Folgekomponenten bevorzugt eine schnelle Abkühlung des Reaktionsgemisches z.B. Entspannung des Reaktionsstroms durchführen. Diese Abkühlung kann in einer möglichen Ausführung durch rasche Entfernung von reaktiven Komponenten z.B. HCl erfolgen. Durch den Druckabbau verdampfen zunächst leichtsiedende Komponenten und kühlen auf Grund der Verdampfungswärme sowie im weiteren Verlauf durch Expansion des Gasvolumen das Gemisch ab. In anderen Ausführungsformen kann die Abkühlung durch Einmischung kühlerer Ströme erfolgen (Quench) oder durch Wärmeabzug über die begrenzenden Wände erfolgen. Möglich ist auch eine Verschaltung der genannten Ausführungsformen. Bevorzugt wird man somit die Reaktionsmischung auf eine Temperatur zwischen 180 und 40 abkühlen, besonders bevorzugt wird man die Reaktionsmischung nach einer Verweilzeit von kleiner als 60 s, bevorzugt zwischen 0,01 und 30 s; besonders bevorzugt zwischen 0,1 s und 2 s bei einer Temperatur von größer als 180°C, bevorzugt zwischen 220 °C und 300 °C, insbesondere zwischen 230 °C und 270 °C auf die Temperatur zwischen 180°C und 40°C abkühlen. Besonders bevorzugt wird man die Reaktionsmischung nach einer Verweilzeit von kleiner als 60 s, bevorzugt zwischen 0,05 und 30 s, besonders bevorzugt zwischen 0,1 s und 2 s bei einer Temperatur von größer als 180°C, bevorzugt zwischen 220 °C und 300 °C, insbesondere zwischen 230 °C und 270 °C in weniger als 1 s um mindestens 50°C abkühlen, indem man die Reaktionsmischung um mindestens 20 bar entspannt.

Das erfindungsgemäße Verfahren bietet aufgrund der getrennten Mischkammern und Reaktionsräumen im Vergleich zu bekannten Verfahren den Vorteil, dass die Reaktionstemperatur sehr gut gesteuert werden kann. Dadurch ist es möglich, die Umsetzung auch bei sehr hohen Reaktionstemperaturen sicher zu beherrschen und eine Überhitzung und damit Zersetzung und Bildung unerwünschter Nebenprodukte zu vermeiden. Bevorzugt verändert sich die Temperatur der Reaktionsmischung während der Verweilzeit von kleiner als 60 s, bevorzugt zwischen 0,05 und 30 s, besonders bevorzugt zwischen 0,1 s und 2 s, bevorzugt bei einer Temperatur von größer als 180°C, bevorzugt zwischen 220 °C und 300 °C, insbesondere zwischen 230 °C und 270 °C um weniger als 20 °C, d.h. die Temperatur des Reaktionsgemisches wird innerhalb des Reaktionsraumes weitgehend konstant gehalten.

Zur Herstellung der Isocyanate könnten allgemein bekannte und zu diesem Zweck übliche Amine eingesetzt werden. Dabei können die Amine einzeln oder in Mischung mit anderen Aminen in den üblichen Qualitäten und Reinheitsstufen verwendet werden. In Frage kommen insbesondere bekannte primäre Amine, Diamine oder Polyamine. Bevorzugt wird man als Amin 2,2'-, 2,4'- und/oder 4,4'-Diamino-diphenylmethan (MDA), 2,4- und/oder 2,6-Toluylendiamin (TDA), 1-Amino-3,3,5-trimethyl-5-amino-methyl-cyclohexan (Isophoron-diamin, IPDA) und/oder Hexamethylendiamin (HDA) einsetzen, besonders bevorzugt 2,2'-, 2,4'- und/oder 4,4'-Diamino-diphenylmethan (MDA) und/oder 2,4- und/oder 2,6-Toluylendiamin (TDA).

Wie bereits eingangs dargestellt erfolgt die Phosgenierung bevorzugt in flüssiger Phase, d.h. dass das Amin bei der Phosgenierung bevorzugt in flüssiger Phase vorliegt.

Das durch die Phosgenierung hergestellte Isocyanat kann durch übliche Verfahren, beispielsweise Destillation, gereinigt werden. Bevorzugt kann in einem ersten Reinigungsvorgang Phosgen und gegebenenfalls Lösungsmittel, bevorzugt weitgehend, besonders bevorzugt vollständig aus dem Reaktionsgemisch der Phosgenierung entfernt werden. Dieser Reinigungsschritt kann bevorzugt durch einen Stripprozess durchgeführt werden. Bei einem solchen Stripprozess kann das Isocyanat in einem Apparat mit großer innerer Oberfläche geleitet und auf dessen Oberfläche verteilt werden, so dass leichtflüchtige Komponenten entweichen können. Es kann sich bei der Apparatur beispielsweise und bevorzugt um einen Fallfilm- oder Dünnschichtverdampfer oder eine gepackte Kolonne geeigneter Auslegung handeln. Inertgase können als Stripmedium eingespeist und/oder Vakuum über die Apparatur angelegt werden. Die Temperaturen während dieses Stripprozesses betragen bevorzugt unter 200°C, besonders bevorzugt 50 bis 190°C. Bevorzugt kann das gewünschte Isocyanat unter bekannten Bedingungen, bevorzugt bei Drücken von 2 bis 50 mbar und Temperaturen von 150 bis 250°C abdestilliert werden.

Besonders bevorzugt führt man die Reinigung des Isocyanats derart durch, dass man bei einer Temperatur von <150°C, bevorzugt 50 bis 149°C, Phosgen, HCl und gegebenenfalls Lösungsmittel beispielsweise in einem bereits beschriebenen Stripprozess, gegebenenfalls unter Vakuum oder Einspeisung von Inertgas, aus dem Isocyanat entfernt, nach bevorzugt vollständiger Entfernung des Phosgens bei einer Temperatur von ≤ 190°C, bevorzugt 150 bis 190°C Lösungsmittel beispielsweise in einem bereits beschriebenen Stripprozess aus dem Isocyanat abtrennt, wobei die Reinigungsschritte mit den bereits beschriebenen Apparaturen durchgeführt werden können, und anschließend das Isocyanat destillativ bei Drücken von 2 bis 50 mbar, bevorzugt 2 bis 20 mbar, und Temperaturen von 150 bis 250°C, bevorzugt 180 bis 230°C abtrennt.

Diese Reinigungsprozesse bieten den Vorteil, dass chlorhaltige Verbindungen, die zu negativen Eigenschaften in dem gewünschten Isocyanat führen, aus dem Isocyanat entfernt werden.

Das nicht umgesetzte überschüssige Phosgen und das Lösungsmittel werden nach der Trennung von den Umsetzungsprodukten bevorzugt in den Stoffumlauf zurückgeleitet.

### Beispiele:

### Herstellung von MDI

180 ml/h einer 22,8 %igen MDA Lösung in MCB und 195 ml/h flüssiges Phosgen wurden durch einen Split and Recombine Micromischer bei einer Temperatur von 245°C und 55 bar im molaren Verhältnis 1/12 MDA/Phosgen geleitet. Die Edukte wurden vor dem Mischen durch jeweils 600mm lange 1/16" Rohre geleitet und vorgewärmt. Nach dem Mischen in diesem 0,02 ml Reaktor wurde der Reaktionstrom kontinuierlich entspannt und in einen Separator geleitet. Im Separator wurde das Produkt mit Stickstoff Phosgen und HCl-frei gestrippt. Anschließend wurde das Produkt noch bei 70 mbar und 50-60°C lösemittelfrei destilliert.

### Herstellung von TDI

210 ml/h einer 17,2 %igen TDA Lösung in MCB und 195ml/h flüssiges Phosgen wurden durch einen T-förmigen laminaren Diffusionsmischer mit einem Volumen von 0,27 ml bei einer Temperatur von 245°C und 55 bar im molaren Verhältnis 1/7 TDA/Phosgen geleitet. Die Edukte wurden vor dem Mischen durch jeweils 600mm lange 1/16" Rohre geleitet und vorgewärmt. Nachdem Mischen in diesem Reaktor wurde der Reaktionsstrom kontinuierlich entspannt und in einen Separator geleitet. Im Separator wurde das Produkt mit Stickstoff Phosgen und HCl-frei gestrippt. Anschließend wurde das Produkt noch bei 70 mbar und 50-60°C lösemittelfrei destilliert.

Die in diesen Beispielen dargestellten Reaktoren können erfindungsgemäß in größerer Anzahl parallel geschaltet werden, wobei die Stoffströme der verschiedenen Reaktoren nach dem Mischen des Amins mit dem Phosgen vereinigt und gemeinsam aufgearbeitet werden.

## Patentansprüche

1. Verfahren zur Herstellung von Isocyanaten durch Phosgenierung von Aminen, **dadurch gekennzeichnet, dass** man Phosgen und Amin in mindestens 2 parallel geschalteten Mischkammern in Kontakt bringt, wobei man als Mischkammern mikrostrukturierte Mischkammern einsetzt.

2. Verfahren nach Anspruch 1, **dadurch gekennzeichnet, dass** man in jede der Mischkammern das Phosgen und das Amin durch getrennte Zuläufe zuführt.

3. Verfahren nach Anspruch 1, **dadurch gekennzeichnet, dass** man als Mischkammern laminare Diffusionsmischer, Multilaminationsmischer, Mikromischer mit strukturierten Wänden, Splitt-Recombine-Mischer, Freistrahlmischer und/oder Düsen einsetzt.

4. Verfahren nach Anspruch 1 oder 3, **dadurch gekennzeichnet, dass** die Mischkammern auf Hasteloy und/oder Glas basieren.

5. Verfahren nach Anspruch 1, **dadurch gekennzeichnet, dass** man einen Phosgenstrom und einen Aminstrom jeweils in eine Mehrzahl von Einzelströmen aufspaltet und die Einzelströme den Mischkammern zuführt, wobei jede Mischkammer mindestens einen Phosgenzulauf und mindestens einen Aminzulauf aufweist.

6. Verfahren nach Anspruch 1, **dadurch gekennzeichnet, dass** man nach dem Mischen des Phosgens mit dem Amin in den Mischkammern die Stoffströme der einzelnen Mischkammern zusammenführt.

7. Verfahren nach Anspruch 1, **dadurch gekennzeichnet**, das man die Umsetzung des Amins mit dem Phosgen bei einer Temperatur von größer als 180 °C durchführt.

8. Verfahren nach Anspruch 1, **dadurch gekennzeichnet, dass** man das Amin mit einer Temperatur von größer als 180 °C in die Mischkammern einführt.

9. Verfahren nach Anspruch 1, **dadurch gekennzeichnet, dass** man das Amin in einem inerten organischen Lösungsmittel mit einem Gewichtsanteil zwischen 5 und 90 Gew.-% bezogen auf das Gesamtgewicht von Amin und Lösungsmittel in die Mischkammern einführt.

10. Verfahren nach Anspruch 1, **dadurch gekennzeichnet, dass** man Amin und Phosgen in einem molaren Verhältnis von Amin zu Phosgen zwischen 1 : 2 und 1 : 20 den Mischkammern zuführt.

11. Verfahren nach Anspruch 1, **dadurch gekennzeichnet, dass** man das Phosgen mit einer Temperatur größer als 180 °C in die Mischkammern einführt.

12. Verfahren nach Anspruch 1, **dadurch gekennzeichnet, dass** man die Umsetzung des Amins mit dem Phosgen bei einem Druck zwischen 20 und 100 bar durchführt.

13. Verfahren nach Anspruch 1, **dadurch gekennzeichnet, dass** die Verweilzeit der Reaktionsmischung bei einer Temperatur von größer als 180 °C kleiner als 60 s beträgt.

14. Verfahren nach Anspruch 1, **dadurch gekennzeichnet, dass** man die Reaktionsmischung nach einer Verweilzeit von kleiner als 60 s bei einer Temperatur von größer als 180 °C auf die Temperatur zwischen 40 °C und 180 °C abkühlt.

15. Verfahren nach Anspruch 1, **dadurch gekennzeichnet, dass** man die Reaktionsmischung nach einer Verweilzeit von kleiner als 60 s bei einer Temperatur von größer als 180 °C in weniger als 1 s um mindestens 50 °C abkühlt, indem man die Reaktionsmischung um mindestens 20 bar entspannt.

16. Verfahren nach Anspruch 14 oder 15, **dadurch gekennzeichnet, dass** sich die Temperatur der Reaktionsmischung während der Verweilzeit von kleiner als 60 s um weniger als 20 °C verändert.

17. Verfahren nach Anspruch 1, **dadurch gekennzeichnet, dass** man als Amin 2,2'-, 2,4'-und/oder 4,4'-Diamino-diphenylmethan (MDA), 2,4- und/oder 2,6-Toluylendiamin (TDA), 1-Amino-3,3,5-trimethyl-5-amino-methyl-cyclohexan (Isophorondiamin, IPDA) und/oder Hexamethylendiamin (HDA) einsetzt.

18. Verfahren nach Anspruch 1, **dadurch gekennzeichnet, dass** das Amin bei der Phosgenierung in flüssiger Phase vorliegt.

19. Verfahren nach Anspruch 1, **dadurch gekennzeichnet, dass** man das Reaktionsprodukt, d.h. das Isocyanat, reinigt, indem man bei einer Temperatur von < 150 °C Phosgen, HCl und gegebenenfalls Lösungsmittel aus dem Isocyanat entfernt, nach Entfernung des Phosgens bei einer Temperatur von ≤ 190 °C Lösungsmittel aus dem Isocyanat abtrennt und anschließend das Isocyanat destillativ bei Drücken von 2 bis 50 mbar und Temperaturen von 150 bis 250 °C abtrennt.

## Claims

1. A process for preparing isocyanates by phosgenation of amines, wherein phosgene and amine are brought into contact in at least 2 mixing chambers connected in parallel and microstructured mixing chambers are used as mixing chambers.

2. The process according to claim 1, wherein the phosgene and the amine are fed through separate inlets into each of the mixing chambers.

3. The process according to claim 1, wherein laminar diffusion mixers, multilamination mixers, micromixers having structured walls, split-recombine mixers, free-jet mixers and/or nozzles are used as mixing chambers.

4. The process according to claim 1 or 3, wherein the mixing chambers are based on Hasteloy and/or glass.

5. The process according to claim 1, wherein a phosgene stream and an amine stream are each split up into a plurality of individual streams and the individual streams are fed into the mixing chambers, with each mixing chamber having at least one phosgene inlet and at least one amine inlet.

6. The process according to claim 1, wherein, after mixing of the phosgene with the amine in the mixing chambers, the streams from the individual mixing chambers are brought together.

7. The process according to claim 1, wherein the reaction of the amine with the phosgene is carried out at a temperature of greater than 180°C.

8. The process according to claim 1, wherein the amine is introduced into the mixing chambers at a temperature of greater than 180°C.

9. The process according to claim 1, wherein the amine is introduced into the mixing chambers as a mixture with an inert gas organic solvent in a proportion by weight in the range from 5 to 90% by weight, based on the total weight of amine and solvent.

10. The process according to claim 1, wherein amine and phosgene are fed into the mixing chambers in a molar ratio of amine to phosgene in the range from 1:2 to 1:20.

11. The process according to claim 1, wherein the phosgene is introduced into the mixing chambers at a temperature of greater than 180°C.

12. The process according to claim 1, wherein the reaction of the amine with the phosgene is carried out at a pressure in the range from 20 to 100 bar.

13. The process according to claim 1, wherein the residence time of the reaction mixture at a temperature of greater than 180°C is less than 60 s.

14. The process according to claim 1, wherein the reaction mixture is cooled after a residence time of less than 60 s at a temperture of greater than 180°C to a temperature in the range from 40°C to 180°C.

15. The process according to claim 1, wherein the reaction mixture is cooled after a residence time of less than 60 s at a temperature of greater than 180°C by at least 50°C in less than 1 s by depressurizing the reaction mixture by at least 20 bar.

16. The process according to claim 14 or 15, wherein the temperature of the reaction mixture changes by less than 20°C during the residence time of less than 60 s.

17. The process according to claim 1, wherein 2,2'-, 2,4'- and/or 4,4'-diamino-diphenylmethane (MDA), 2,4- and/or 2,6-toluenediamine (TDA), 1-amino-3,3,5-trimethyl-5-aminomethylcyclohexane (isophoronediamine, IPDA) and/or hexamethylenediamine (HDA) are used as amine.

18. The process according to claim 1, wherein the amine is present in the liquid phase during the phosgenation.

19. The process according to claim 1, wherein the reaction product, i.e. the isocyanate, is purified by removing phosgene, HCl and if appropriate solvent from the isocyanate at a temperature of < 150°C and, after removal of the phosgene, at a temperature of ≤ 190°C, separating off solvent from the isocyanate and subsequently separating off the isocyanate by distillation at pressures of from 2 to 50 mbar and temperatures of from 150 to 250°C.

## Revendications

1. Procédé pour la préparation d'isocyanates par phosgénation d'amines, **caractérisé en ce qu'**on met en contact le phosgène et l'amine dans au moins 2 chambres de mélange commutées en parallèle, en utilisant, comme chambres de mélange, des chambres de mélange microstructurées.

2. Procédé selon la revendication 1, **caractérisé en ce qu'**on introduit le phosgène et l'amine dans chacune des chambres de mélange via des alimentations séparées.

3. Procédé selon la revendication 1, **caractérisé en ce qu'**on utilise comme chambres de mélange des mélangeurs à diffusion laminaire, des mélangeurs à flux laminés multiples, des micromélangeurs à parois structurées, des mélangeurs à fractionnement-recombinaison, des mélangeurs à jets libres et/ou des gicleurs.

4. Procédé selon la revendication 1 ou 3, **caractérisé en ce que** les chambres de mélange sont à base d'Hasteloy et/ou de verre.

5. Procédé selon la revendication 1, **caractérisé en ce qu'**on dissocie un flux de phosgène et un flux d'amine à chaque fois en une multitude de flux individuels et on introduit les flux individuels dans des chambres de mélange, chaque chambre de mélange présentant au moins une alimentation de phosgène et au moins une alimentation d'amine.

6. Procédé selon la revendication 1, **caractérisé en ce qu'**on réunit, après le mélange du phosgène avec l'amine dans les chambres de mélange, les flux de matières de différentes chambres de mélange.

7. Procédé selon la revendication 1, **caractérisé en ce qu'**on réalise la transformation de l'amine avec le phosgène à une température supérieure à 180°C.

8. Procédé selon la revendication 1, **caractérisé en ce qu'**on introduit l'amine à une température supérieure à 180°C dans les chambres de mélange.

9. Procédé selon la revendication 1, **caractérisé en ce qu'**on introduit l'amine dans un solvant organique inerte à une proportion pondérale entre 5 et 90% en poids par rapport au poids total de l'amine et du solvant dans les chambres de mélange.

10. Procédé selon la revendication 1, **caractérisé en ce qu'**on introduit l'amine et le phosgène dans un rapport molaire d'amine à phosgène entre 1:2 et 1:20 dans les chambres de mélange.

11. Procédé selon la revendication 1, **caractérisé en ce qu'**on introduit le phosgène à une température supérieure à 180°C dans les chambres de mélange.

12. Procédé selon la revendication 1, **caractérisé en ce qu'**on réalise la transformation de l'amine avec le phosgène à une pression entre 20 à 100 bars.

13. Procédé selon la revendication 1, **caractérisé en ce que** le temps de séjour du mélange réactionnel à des températures supérieures à 180°C est inférieur à 60 secondes.

14. Procédé selon la revendication 1, **caractérisé en ce qu'**on refroidit le mélange réactionnel, après un temps de séjour inférieur à 60 s à une température supérieure à 180°C, à la température entre 40°C et 180°C.

15. Procédé selon la revendication 1, **caractérisé en ce qu'**on refroidit le mélange réactionnel, après un temps de séjour inférieur à 60 s à une température supérieure à 180°C, en moins de 1 seconde d'au moins 50°C **en ce qu'**on détend le mélange réactionnel d'au moins 20 bars.

16. Procédé selon la revendication 14 ou 15, **caractérisé en ce que** la température du mélange réactionnel change de moins de 20°C pendant le temps de séjour de moins de 60 secondes.

17. Procédé selon la revendication 1, **caractérisé en ce qu'**on utilise, comme amine, le 2,2'-diaminodiphénylméthane, le 2,4'-diaminodiphénylméthane et/ou le 4,4'-diaminodiphénylméthane (MDA), la 2,4-toluylènediamine et/ou la 2,6-toluylènediamine (TDA), le 1-amino-3,3,5-triméthyl-5-aminométhylcyclohexane (isophoronediamine, IPDA) et/ou l'hexaméthylènediamine (HDA).

18. Procédé selon la revendication 1, **caractérisé en ce que** l'amine, lors de la phosgénation, se trouve dans la phase liquide.

19. Procédé selon la revendication 1, **caractérisé en ce qu'**on purifie le produit de réaction, c'est-à-dire l'isocyanate, en éliminant, à une température < 150°C, le phosgène, le HCl et le cas échéant le solvant de l'isocyanate, après séparation du phosgène à une température ≤ 190°C le solvant est séparé de l'isocyanate, puis on sépare l'isocyanate par distillation à des pressions de 2 à 50 mbars et des températures de 150 à 250°C.
